# EUROPÄISCHE PATENTANMELDUNG

(11) **EP 0 943 294 A2**
(43) Veröffentlichungstag der Anmeldung: **22.09.1999**
(21) Anmeldenummer: 99105398.4
(22) Anmeldetag: 16.03.1999
(51) Int. Cl.: A61B 19/00

(54) **Vorrichtung zur Entfernung von Gewebeproben von einem medizinischen Instrument**

(30) Priorität: 19.03.1998 DE 19812099; 26.03.1998 DE 19813483
(71) Anmelder: B. BRAUN MELSUNGEN AG, 34212 Melsungen (DE)
(72) Erfinder: Frimberger, Eckart, Dr., 80804 München (DE)
(74) Vertreter: Körber, Wolfhart, Dr. rer.nat.

(57) **Zusammenfassung**

Eine Vorrichtung zur Entfernung von Gewebeproben von einem medizinischen Instrument, beispielsweise einer endoskopischen Sonde, weist ein Gehäuse (1), einen Vibrationsgenerator (2, 10) zur Erzeugung von Vibrationsbewegungen, eine mit dem Vibrationsgenerator (2) gekoppelte Vibrationsplatte (3) mit einem Eingriffsabschnitt (4) zur Übertragung der Vibrationsbewegung auf das medizinische Instrument (15) und eine Schaltereinrichtung zum Ein- und Ausschalten des Vibrationsgenerators (2, 10) auf. Durch die Vibrationsbewegungen löst sich die Gewebeprobe von dem medizinischen Instrument (15) und fällt beispielsweise in ein Probengefäß (11). Die Gewebeprobe kann so einfach und sicher von dem medizinischen Instrument (15) entfernt werden.

## Beschreibung

Die Erfindung betrifft eine Vorrichtung zur Entfernung von Gewebeproben von einem medizinischen Instrument und eine als Sonde ausgebildetes Instrument.

Im Rahmen der endoskopischen Diagnostik werden häufig aus verschiedenen Organen, beispielsweise dem Magen-Darm-Trakt mit Hilfe geeigneter Instrumente Gewebsproben entnommen (Biopsie). Die dafür verwendete Sonde wird dabei in den Instrumentierkanal des Endoskops eingeführt und kann mittels eines durch den Instrumentierkanal geführten Bowdenzuges von außerhalb des Körpers des Patienten betätigt werden. Ein vorzugsweise dafür eingesetztes Instrument ist die Biopsie-Zange im wesentlichen bestehend aus einem Bowdenzug mit Griff und einem Zangenmaul. Durch Griffbetätigung kann das Zangenmaul geöffnet und geschlossen werden, wodurch ein Gewebspartikel vom Gewebe entfernt und festgehalten werden kann. Der Endoskopiker führt die Biopsie-Zange durch den Instrumentierkanal des Endoskops an den Einsatzort im Körper, die assistierende Schwester betätigt die Zange zur Gewebsentnahme (Biopsie). Nach der Biopsie zieht der Endoskopiker die Zange aus dem Endoskop zurück, die Schwester hält mit der einen Hand den Griff der Biopsie-Zange, führt mit der anderen Hand das Zangenmaul in ein Probengläschen, wo die Probe durch Schütteln der Zange entfernt wird und in das mit konservierender Flüssigkeit gefüllte Gläschen fällt.

Da die zumeist kleinen, im wäßrigen Gewebeumfeld entnommenen Proben gut an dem Entnahmewerkzeug haften, tritt häufig das Problem auf, die Gewebeprobe wieder von dem medizinischen Instrument zu entfernen. Dies geschieht häufig durch heftiges Schütteln des Instruments in einem mit konservierender Flüssigkeit gefüllten Probengefäß. Dies ist mühsam und beansprucht in unerwünschter Weise die Aufmerksamkeit des Arztes oder der assistierenden Krankenschwester. Außerdem kann das Schütteln zu einem Überschwappen der konservierenden Flüssigkeit führen. Die Gewebsprobenfreisetzung ist dann zusätzlich erschwert, wenn die Gewebsprobe sehr klein und daher schlecht sichtbar ist.

Der vorliegenden Erfindung liegt daher die Aufgabe zugrunde, eine Vorrichtung und ein medizinisches Instrument vorzuschlagen, die eine einfache und zuverlässige Entfernung von Gewebeproben oder Gewebepartikeln von einem medizinischen Instrument ermöglicht.

Gelöst wird die Aufgabe erfindungsgemäß durch eine Vorrichtung zur Entfernung von Gewebeproben von einem medizinischen Instrument, die ein Gehäuse, einen Vibrationsgenerator zur Erzeugung von Vibrationsbewegungen, eine mit dem Vibrationsgenerator gekoppelte Vibrationsplatte mit einem Eingriffsabschnitt zur Übertragung der Vibrationsbewegung auf das medizinische Instrument und eine Schaltereinrichtung zum Ein- und Ausschalten des Vibrationsgenerators aufweist.

Das medizinische Instrument wird in Kontakt mit dem Eingriffsabschnitt der vibrierenden Platte gebracht, so daß sich die Vibrationsbewegung auf das medizinische Instrument übertragt. Die von dem Vibrationsgenerator erzeugten Vibrationsfrequenzen sind wesentlich höher als die durch manuelles Schütteln erreichbaren Frequenzen. So kann die Gewebeprobe sicher von dem medizinischen Instrument entfernt werden.

Die Schaltereinrichtung zum Ein- und Ausschalten des Vibrationsgenerators wird vorzugsweise durch Einführen des medizinischen Instruments in den Eingriffsabschnitt der Vibrationsplatte betätigt. Dies kann durch einen Druckschalter erfolgen, der einen mit dem Instrument auf die Vibrationsplatte ausgeübten Druck erfaßt und dann den Vibrationsgenerator einschaltet. Eine andere Möglichkeit ist eine Lichtschranke oder ein Bewegungssensor zur Erfassung des medizinischen Instrumentes. Weist das medizinische Instrument einen betätigbaren Greifmechanismus, beispielsweise ein öffen- und schließbares Zangenmaul auf, so kann der Schalter für den Vibrationsgenerator auch mit einer Betätigung des Greifmechanismus des Instruments gekoppelt sein. Wird beispielsweise das Zangenmaul einer Endoskopiezange geöffnet, so geht gleichzeitig der Vibrationsgenerator in Betrieb.

Vorzugsweise weist die Vibrationsplatte zwei Ausnehmungen auf und ist mittels zweier Kopplungselemente entfernbar mit dem Gehäuse verbunden. Eines der Kopplungselemente ist vorzugsweise mit dem Vibrationsgenerator verbunden und überträgt die Vibrationsbewegungen auf die Vibrationsplatte. Das andere Kopplungselement kann fest oder bewegbar mit dem Gehäuse verbunden sein.

Der Eingriffsabschnitt für das medizinische Instrument kann dem jeweils verwendeten Instrument angepaßt ausgebildet sein. Denkbar ist ein keilförmiger Eingriffsabschnitt oder ein elastisches Element mit einer Öffnung zum Einführen des medizinischen Instruments. Das elastische Element hat den Vorteil, daß die Vibrationsbewegung sicher auf Instrumente bzw. Sonden mit verschiedenen Außenabmessungen und Formen übertragen werden können.

Eine Weiterbildung der Erfindung weist zusätzlich eine bewegbare Schiene mit Aussparungen für Probengefäße auf, die im Abstand der Probengefäße einrastbar ist, so daß sich das medizinische Instrument immer über einem Probengefäß befindet. So wird sichergestellt, daß die Gewebeprobe immer in das dafür vorgesehene Probengefäß fällt. Um auch in dunkler Umgebung, die beispielsweise bei endoskopischen Untersuchungen üblich ist, die Probe in dem Probengefäß sicher erkennen zu können, kann eine Beleuchtungseinrichtung zur Beleuchtung des Probengefäßes vorgesehen sein.

Der Vibrationsgenerator kann vorzugsweise ein Elektromotor mit angekoppeltem Exzenter sein.

Der Erfindung liegt weiter die Aufgabe zugrunde, ein medizinisches Instrument, insbesondere eine Sonde zu schaffen, welche ein sicheres Inbetriebsetzen des Vibrationsgenerators gestattet, wobei im Zeitpunkt des Einschalten des Vibrators sichergestellt ist, daß die abzuschüttelnde Probe bzw. deren Greifmechanismus die korrekte Position über der Mündung des die Probe aufnehmenden Gläschens einnimmt.

Diese Aufgabe wird durch die Merkmale des Anspruches 1 gelöst. Zweckmäßige Ausgestaltungen sind in den Unteransprüchen gekennzeichnet.

Durch die Anordnung der Schaltmittel in einem vorgegebenen Abstand an der Sonde ist es sichergestellt, daß im Zeitpunkt des Einschaltens des Vibrationsgerätes der Greifmechanismus mit der abzuschüttelnden Probe stets in einer Lage über dem aufnehmenden Gläschen ist, daß einerseits die abzuschüttelnde Probe sicher tatsächlich in das Gläschen gelangt und andererseits der Greifmechanismus nicht so tief in das Gläschen oder die darin befindliche Flüssigkeit eintaucht, daß der Abschüttelvorgang behindert wird.

Im folgenden wird die Erfindung anhand bevorzugter Ausführungsbeispiele unter Bezugnahme auf die Zeichnung erläutert, in der
- Figur 1: eine schematische Seitenansicht eines bevorzugten Ausführungsbeispiels der erfindungsgemäßen Vorrichtung ist;
- Figur 2: eine Stirnansicht der Vorrichtung von Figur 1 ist;
- Figur 3: eine Aufsicht auf ein erstes Ausführungsbeispiel einer Vibrationsplatte der Vorrichtung von Figur 1 ist;
- Figur 4: eine Aufsicht auf ein zweites Ausführungsbeispiel einer Vibrationsplatte ist.
- Figur 5: eine Ansicht entsprechend Figur 1 mit einem anderen Instrument und Ultraschallvibrator.
- Figur 6: schematisch drei Ausführungen a), b), c) jeweils eines distalen Endes einer Instrumenten-Sonde mit Schaltmittel.

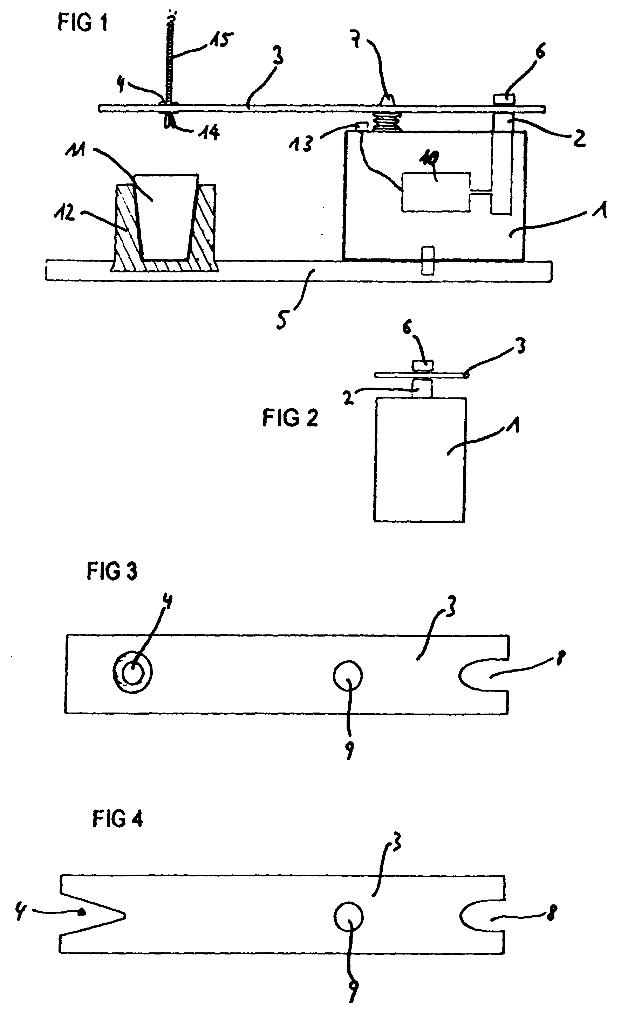

Figur 1 zeigt die erfindungsgemäße Vorrichtung zur Entfernung von Gewebeproben schematisch in Seitenansicht. Auf einer Grundplatte 5 ist ein Gehäuse 1 befestigt, in der sich ein Elektromotor 10 mit daran gekoppeltem Exzenter 2 befindet. Die Vibrationsbewegung wird von dem Exzenter 2 mittels eines ersten Kopplungselements 6 auf eine Vibrationsplatte 3 übertragen, die zusätzlich mit einem zweiten Kopplungselement 7 gegenüber dem Gehäuse 1 gelagert ist. Bei dem gezeigten Ausführungsbeispiel weist die Vibrationsplatte, wie in Figur 3 und 4 gezeigt ist, eine hintere Ausnehmung 8 und ein Loch 9 auf. Die Vibrationsplatte kann so auf die konisch ausgeformte Spitze des Kopplungselements 7 aufgelegt und durch Einschieben in die zwischen Exzenter 2 und erstem Kopplungselement 6 gebildete Nut fixiert werden. Die vorzugsweise aus Kunststoff ausgebildete Vibrationsplatte 3 kann so leicht am Gehäuse 1 fixiert und wieder entfernt werden. Es sind jedoch auch andere Befestigungsvarianten denkbar, bei denen die Vibrationsbewegung von dem Exzenter auf die Vibrationsplatte übertragen wird.

Im vorderen Bereich weist die Vibrationsplatte 3 einen Eingriffsabschnitt 4 für das medizinische Instrument 15, beispielsweise eine Biopsiezange 14 oder dergleichen auf. Der Eingriffsabschnitt kam der Form und Größe des medizinischen Instruments entsprechend ausgebildet sein. In Figur 1 und Figur 3 ist ein gestrichelt dargestelltes ringförmiges elastisches Element 4 gezeigt, das das Einführen von Instrumenten verschiedener Größe und Form ermöglicht, wobei jeweils eine sichere Übertragung der Vibrationsbewegung auf das Instrument sichergestellt ist. Der Innendurchmesser des ringförmigen Elements 4 kann so gewählt sein, daß die Vibrationsplatte durch eine am medizinischen Instrument 15 angebrachte ringförmige äußere Abstandsmarkierung nach unten gedrückt werden kann, wodurch die Vibrationsbewegung aktiviert wird. In Figur 4 ist als eine mögliche Alternative ein keilförmiger Eingriffsabschnitt 4 gezeigt, in den das Instrument eingeschoben und mit der Hand fixiert wird.

Die Vorrichtung zur Entfernung von Gewebeproben weist einen Schalter zum Ein- und Ausschalten des Elektromotors 10 auf. Dabei kann ein Schalter verwendet werden, der durch Niederdrücken der Vibrationsplatte 3 durch das medizinische Instrument betätigt wird. Der Schalter kann zum Beispiel mit dem Kopplungselement 7 verbunden sein. Eine andere Möglichkeit ist in Figur 1 gezeigt. Ein Sensor 13, beispielsweise eine Lichtschranke oder ein Bewegungssensor erfaßt das medizinische Instrument 15 und aktiviert den Elektromotor 10 zum Antrieb des Exzenters 2. Eine weitere Möglichkeit ist, den Schalter mit einer Betätigung des medizinischen Instruments 15 zu koppeln. Wird beispielsweise das Zangenmaul einer Biopsiezange geöffnet, so schaltet gleichzeitig der Schalter den Elektromotor 10 ein.

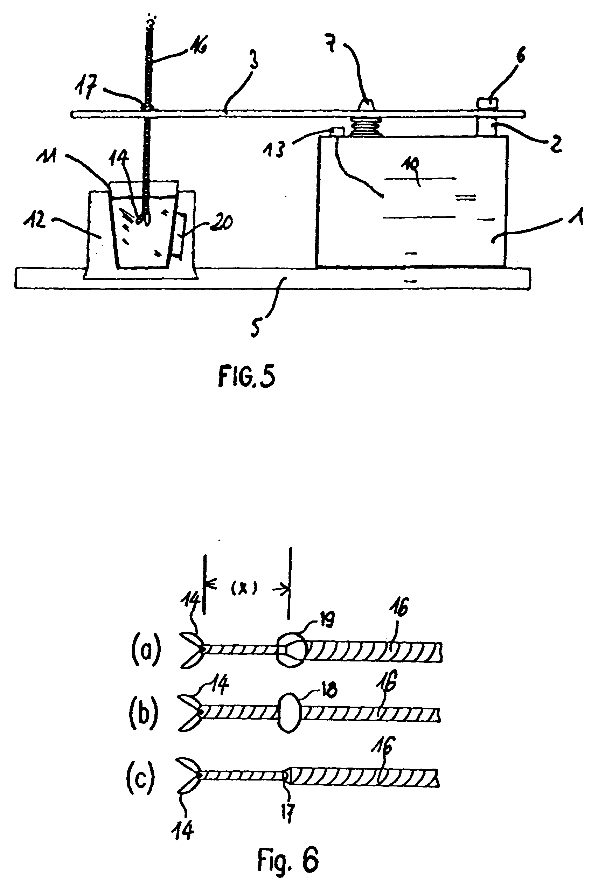

Aus Figur 5 ist ein medizinisches Instrument in Form einer Sonde 16 gezeigt, die in einem vorgegebenen Abstand (x) (Figur 6) von der Gewebeprobe bzw. Biopsiezange 14 ein Schaltmittel 17 aufweist, so daß sich beim in Betrieb setzen der Vibrationsplatte 3 und/oder des Ultraschallgenerators 20 die Gewebeprobe in einer vorgegebenen Höhe in dem Probengefäß 11 befindet.

Das in Figuren 1 und 5 gezeigte Ausführungsbeispiel der erfindungsgemäßen Vorrichtung weist weiterhin eine bewegbare Schiene 12 mit Aussparungen für Probengefäße 11 auf. Die Schiene ist senkrecht zur Längsrichtung der Vibrationsplatte 3 bewegbar und rastet in Positionen im Abstand der Probengefäße ein, so daß sich in jeder Einrastposition immer ein Probengefäß 11 unterhalb des Eingriffsabschnitts 4 der Vibrationsplatte 3 befindet. So kann man das Probengefäß mit einem Handgriff sicher unter dem medizinischen Instrument 15, 16 positionieren. Die Schiene 12 kann auch motorgetrieben sein. Die Probe kann so definiert in das zugehörige Probengefäß freigesetzt werden.

Bei den in Figur 6 gezeigten Ausführungsformen der Schaltmittel an der Sonde 16 für eine mechanische Auslösung der Schaltung bei einem Vibrationsgerät entsprechend Figur 5 ist das Schaltmittel in einem vorgegebenen Abstand von dem Greifmechanismus 14 als sich radial erstreckende Anschlagstufe 17 ausgebildet oder als ringförmiger Vorsprung 18 bzw. als angebrachtes Kügelchen 19 ausgebildet. Entscheidend ist, daß diese Schaltmittel, die bei entsprechender Ausbildung des Vibrationsgerätes auch einen Schalter optisch beinflussende Mittel sein können, einen vorgegebenen Abstand von dem Greifmechanismus 14 der Sonde haben, wobei der vorgegebene Abstand an die Position der Schaltmittel an dem Vibrationsgerät in ihrer Lage relativ zur Position des Probengefäßes 11 angepaßt ist.

Die Erfindung ermöglicht so eine einfache und sichere Entfernung von Gewebeproben von einem medizinischen Instrument. Die Handhabung für den Arzt oder die assistierende Krankenschwester ist weitestmöglich automatisiert. Darüberhinaus stellt die erfindungsgemäße Vorrichtung eine schonende Behandlung der Probe sicher.

## Patentansprüche

1. Vorrichtung zur Entfernung von Gewebeproben von einem medizinischen Instrument (15), aufweisend
ein Gehäuse (1),
ein Vibrationsgenerator (2, 10) zur Erzeugung von Vibrationsbewegungen,
eine mit dem Vibrationsgenerator (2, 10) gekoppelte Vibrationsplatte (3) mit einem Eingriffsabschnitt (4) zur Übertragung der Vibrationsbewegung auf das medizinische Instrument (15),
eine Schaltereinrichtung zum Ein- und Ausschalten des Vibrationsgenerators (2, 10).

2. Vorrichtung nach Anspruch 1, **dadurch gekennzeichnet,** daß die Schaltereinrichtung ein mit dem medizinischen Instrument (15) oder manuell betätigter Druckschalter ist.

3. Vorrichtung nach Anspruch 1 oder 2, **dadurch gekennzeichnet,** daß die Schaltereinrichtung ein automatischer Schalter ist, der ein in dem Eingriffsabschnitt (4) der Vibrationsplatte (3) befindliches medizinisches Instrument (5) erfaßt.

4. Vorrichtung nach Anspruch 3, **dadurch gekennzeichnet,** daß der automatische Schalter eine Lichtschranke oder einen Bewegungssensor (13) aufweist.

5. Vorrichtung nach einem der Ansprüche 1 bis 4, **dadurch gekennzeichnet,** daß die Vibrationsplatte (3) zwei Ausnehmungen (8, 9) aufweist und mittels zweier Kopplungselemente (6, 7) entfernbar mit dem Gehäuse (1) verbunden ist.

6. Vorrichtung nach Anspruch 5, **dadurch gekennzeichnet,** daß ein erstes (6) der Kopplungselemente an den Vibrationsgenerator (2, 10) angekoppelt ist und zur Übertragung der Vibrationsbewegungen vom Vibrationsgenerator (2) auf die Vibrationsplatte (3) ausgebildet ist, und ein zweites (7) der Kopplungselemente fest oder beweglich mit dem Gehäuse (1) verbunden ist.

7. Vorrichtung nach Anspruch 6, **dadurch gekennzeichnet,** daß das zweite Kopplungselement (6) mit einem elastischen Schlauch, O-Ring, Dichtungsring oder Faltenbalg gegenüber dem Gehäuse (1) abgedichtet ist.

8. Vorrichtung nach einem der Ansprüche 1 bis 7, **dadurch gekennzeichnet,** daß der Eingriffsabschnitt (4) der Vibrationsplatte (3) keilförmig, bogenförmig oder als Federelement ausgebildet ist.

9. Vorrichtung nach einem der Ansprüche 1 bis 7, **dadurch gekennzeichnet,** daß der Eingriffsabschnitt (4) der Vibrationsplatte ein elastisches Element mit einer Öffnung für das medizinische Instrument (15) aufweist.

10. Vorrichtung nach einem der Ansprüche 1 bis 9, **gekennzeichnet durch** eine über eine Bodenplatte (5) mit dem Gehäuse (1) verbundene Aufnahme (12) mit mindestens einer Aussparung zur Aufnahme von Probengefäßen (11).

11. Vorrichtung nach Anspruch 10, **dadurch gekennzeichnet,** daß die Aufnahme als bewegbare Schiene (12) ausgebildet ist, die in verschiedenen Positionen im Abstand der Probengefäße (11) einrastbar ist, so daß sich in jeder eingerasteten Position der Schiene (12) eine Aussparung für ein Probengefäß (11) unterhalb des Eingriffsabschnitts (4) der Vibrationsplatte (3) befindet.

12. Vorrichtung nach Anspruch 10 oder 11, **gekennzeichnet durch** eine Beleuchtungseinrichtung für das Probengefäß (11).

13. Vorrichtung nach Anspruch 12, **dadurch gekennzeichnet,** daß die Beleuchtungsvorrichtung an die Schaltereinrichtung des Vibrationsgenerators gekoppelt ist.

14. Vorrichtung nach einem der Ansprüche 10 bis 13, **dadurch gekennzeichnet,** daß die bewegbare Schiene (12) motorbetrieben ist.

15. Vorrichtung nach einem der Ansprüche 3 bis 14, **dadurch gekennzeichnet,** daß der automatische Schalter zur Betätigung des Vibrationsgenerators (2, 10) an eine Betätigung des medizinischen Instruments (15) gekoppelt ist.

16. Vorrichtung nach einem der Ansprüche 1 bis 15, **dadurch gekennzeichnet,** daß der Vibrationsgenerator (2, 10) einen Elektromotor (10) mit einem Exzenter (2) aufweist.

17. Vorrichtung nach mindestens einem der Ansprüche 3 bis 16, **dadurch gekennzeichnet,** daß das medizinische Instrument (16) in einem vorgegebenen Abstand von dem die Gewebeprobe aufnehmenden Mechanismus (14) ein mechanisch und/oder optisch erfaßbares Schaltmittel (17, 18, 19) zum Betätigen des automatischen Schalters der Vibrationsplatte (3) aufweist, wobei der Abstand derart ist, daß sich die Gewebeproben in einer gewünschten Höhenlage innerhalb des Probengefäßes (11) befindet.

18. Vorrichtung nach Anspruch 17, **dadurch gekennzeichnet,** daß das Schaltmittel ein Kalibriervorsprung am Mantel der Sonde ist.

19. Vorrichtung nach Anspruch 17 oder 18, **dadurch gekennzeichnet,** daß das Schaltmittel eine sich radial von der Außenseite der Sonde erstreckende Anschlagstufe (17) ist.

20. Vorrichtung nach Anspruch 17 oder 18, **dadurch gekennzeichnet,** daß das Schaltmittel ein auf der Sonde angebrachter ringförmiger oder nasenartiger Vorsprung (18, 19) ist.

21. Vorrichtung nach einem der Ansprüche 10 bis 20, **dadurch gekennzeichnet,** daß im Bereich der Aufnahme (12) ein Ultraschallgenerator (20) zum Einbringen eines Ultraschallfeldes in die Flüssigkeit in dem Probengefäß (11) vorgesehen ist, wobei der Ultraschallgenerator (20) durch die Schaltmittel geschaltet wird.
